# EUROPEAN PATENT SPECIFICATION

(11) **EP 4 680 330 B1**
(45) Date of publication and mention of the grant of the patent: **15.04.2026**
(21) Application number: 24759104.3
(22) Date of filing: 20.08.2024
(51) Int. Cl.: A61N 2/04, A61N 2/02

(54) **ELECTROMAGNETIC APPLICATOR SYSTEM**
ELEKTROMAGNETISCHES APPLIKATORSYSTEM
SYSTÈME APPLICATEUR ÉLECTROMAGNÉTIQUE

(30) Priority: 21.08.2023 DE 102023122274
(43) Date of publication of application: 21.01.2026
(73) Proprietor: Gleim, Niki Reiner, 9485 Nendeln (LI)
(72) Inventor: DOBLER, Karl, 6845 Hohenems (AT); GLEIM, Niki Reiner, 9485 Nendeln (LI)
(74) Representative: Epping - Hermann - Fischer
(86) International application number: PCT/EP2024/073310
(87) International publication number: WO 2025/040664

(56) References cited:
- WO-A1-2022/133360
- US-B2- 10 806 942

## Description

### Technical field of the invention

The invention relates to an electromagnetic applicator system which can be used, for example, as a device for magnetic field therapy.

### Prior art

It is known to treat various physical complaints in living beings with pulsed electromagnetic field (PEMF) therapy.

In conventional devices, coils, such as Helmholtz coils, are subjected to high-intensity pulsed signals for this purpose, which are generated by a stationary control unit. Due to the high intensities and the associated high currents required, high losses occur in such conventional devices, which must be dissipated as thermal energy via special cooling devices such as a water-cooling system. The devices can only be supplied via the power supply network. In addition, such devices cannot be used flexibly.

In some conventional devices, the problem of heat loss is countered by greatly reducing the corresponding intensities of the currents of the pulsed signals and the resulting magnetic field. However, this leads to a reduced effectiveness of the therapy.

Document US 10,806,942 B2 discloses a system and method for applying a low strength, low frequency magnetic field therapy to biological tissues. A coil is excited with a low frequency oscillating current, e.g., 10-1000 Hz. The coil is, e.g., 5-200 turns, having a diameter of 2-20 mm, and produces a magnetic field strength of about 0.01-5 mTelsa at a distance of 1 cm from the coil, or a cover over the coil, into the tissue. The coil may be in mineral housing with a simple filter, and TRRS-type audio jack.

Document WO 2022/133360 discloses a method for delivery of PEMF energy therapy to modulate inflammation using high-frequency current pulsed through one or more coils.

### Objectives and problem solution

An object to be achieved is to provide an improved concept for magnetic field therapy which is characterized by high efficiency and flexibility even at high power.

This object is achieved with the independent claim, with advantageous further developments being specified in the dependent claims.

The improved concept is based on the idea of making an electromagnetic applicator system so efficient by minimizing the power loss, in particular in the coils, that it has only a low energy requirement despite high pulse power and can be supplied from a battery pack. It was found that in the frequency range in which the pulse signals are generated, a significant reduction in power loss can be achieved by using high-frequency stranded wires. Nevertheless, short-term pulse energies in the megawatt range can be achieved.

For example, one embodiment of an electromagnetic applicator system according to the improved concept comprises a housing containing a control unit, a battery pack for supplying the control unit and externally accessible plug connections. The applicator system also comprises at least one applicator, which comprises at least one coil that is connectable to the plug connections via a supply line with an at least two-pole plug connection. According to the improved concept, the at least one coil and the supply line are formed by means of a high-frequency stranded wire.

The control unit is set up to generate a sequence of electrical pulse signals from energy stored in the battery pack for output via the plug connections, so that the at least one applicator generates an electromagnetic field when it is connected to the plug connections. Each pulse signal has a maximum pulse power in the range from 100 kW to 10 MW with a pulse length in the range from 100 µs to 10 ms. This means that the pulse power is higher than with conventional devices of this type.

The applicator system is suitable and set up for PEMF treatment, for example.

While the applicator used in conventional devices is permanently connected to a signal generator or can only be detached from it with increased effort, the improved concept provides for various applicators to be easily connected to and detached from the control unit via the plug connections. This results in increased flexibility. For example, the plug connections are safety-tested and can withstand the high currents and voltages required for the maximum pulse power. For example, the plug connections contain safety mechanisms that prevent current and voltage from being applied to the plug connections unless the applicator's plug connection is connected. This ensures a high level of product safety for the user.

For example, each pulse signal has a maximum current in the range from 100 A to 10 kA. In addition, each pulse signal has a maximum voltage in the range of 100 V to 10 kV, for example. Due to the short pulse lengths and the high pulse powers or high maximum currents required, it is desirable for the control unit to be able to generate a high gradient in the current waveform. For example, each pulse signal has a current waveform such that a magnetic field change with a gradient in the order of 1 T/ms results in the applicator.

For example, the average maximum value in current or power is regulated via the voltage.

In various embodiments, the sequence of pulse signals contains, for example, 5 to 30 electrical pulse signals per second. Such a low frequency of pulse signals has been found to be favorable for therapy.

In various embodiments, the applicator system is set up for a respective application duration of up to 20 minutes. For example, such a treatment duration of up to 20 minutes can be completely supplied from the battery pack, in particular without having to rely on a mains supply and/or charging the battery pack in the meantime.

For example, the applicator system has a passive cooling system. In particular, the applicator system is free of fan cooling and water cooling. In contrast, conventional applicators often require water cooling of the coils in order to dissipate the high energy losses. As fewer losses occur in the applicator, the power requirement in the control unit is also lower, so that the losses in the control unit are also reduced. The control unit can therefore be operated with passive cooling without the use of fans. Fanless operation is also more pleasant for a user of the applicator system, as no disturbing noises are generated. Alternatively or additionally, if required, the control unit is cooled with one or more virtually silent fans that generate a noise level of less than 17 dB(A). Water cooling can be dispensed with in any case.

In various embodiments, the high-frequency stranded wire has several thousand conductors. The large number of conductors significantly reduces the skin effect, which plays a major role in the generation of heat loss.

The battery pack has a capacity in the range of 200 Wh to 400 Wh, for example. This enables longer operating times for the applicator system, even at high pulse outputs. The battery pack can be recharged, for example from a mains power supply, although this is not an immediate requirement for operating the applicator system.

In various embodiments, the housing also comprises a control panel for operating the control unit. For example, the control panel is designed as a display, in particular as a touch-sensitive display.

In various embodiments, the housing is formed by a carrying case containing the battery pack and the control unit. In addition, in some embodiments, for example, one or more applicators can be stored and transported in the case, so that a high degree of portability of the applicator system is ensured.

In some embodiments, the case also contains the aforementioned control panel, whereby this is arranged somewhat in a lid of the case. For example, the applicator system is operated with the lid of the case open so that the user can operate the applicator system via the control panel in the lid.

While only the basic properties of the pulse signals or the sequence of pulse signals have been described here, different parameterizations of the sequence or the pulse signals can be carried out in different operating modes.

In various embodiments, the applicator system can also have several different types of applicators, which can be connected to the housing and the control unit to treat a specific part of the body, depending on the application. For example, one or more of these applicators are flexible and equipped with corresponding straps or the like in order to attach the respective applicator to the desired body part. The various applicators can differ, for example, in size, the number of coils or the like.

The improved concept is explained in more detail below with reference to the drawings using examples of embodiments. Similar elements or elements with the same functions are labelled with the same reference signs. Therefore, a repeated explanation of individual elements is not necessary.

In the drawings
- Figure 1: shows a schematic representation of an embodiment of an electromagnetic applicator system,
- Figure 2: shows a basic time diagram of a sequence of pulse signals,
- Figures 3 to 6: show various embodiments of applicators for use with the electromagnetic applicator system, and
- Figures 7 and 8: show representations of an embodiment of an electromagnetic applicator system.

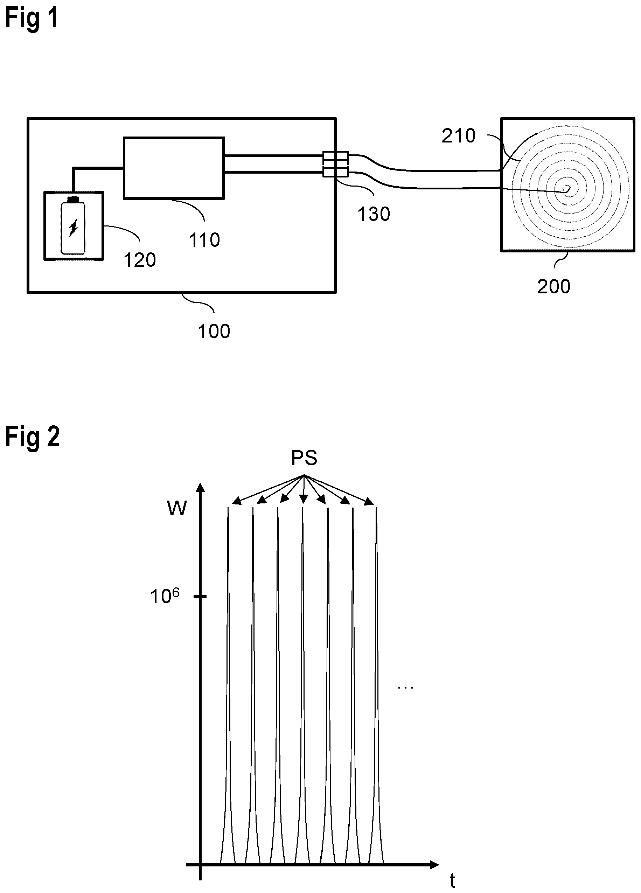

Figure 1 shows a schematic representation of an embodiment of an electromagnetic applicator system with a housing 100, which contains a control unit 110, a battery pack 120 for supplying the control unit 110 and plug connections 130 accessible from the outside. In particular, the plug connections are led from the inside of the housing 100 to the outside.

The applicator system further comprises at least one applicator 200, which comprises at least one coil 210, which can be connected to the plug connections 130 via a supply line with an at least two-pole plug connection. In this way, the electrical connection can be established between the applicator 200 and the control unit 110, which is connected on the output side inside the housing to the plug connections 130.

The at least one coil 210 and the supply line from the plug connections 130 to the coil 210 are formed by means of a high-frequency stranded wire. A plurality of insulated individual conductors is brought together to form a strand of electrically parallel conductors. For example, high-frequency stranded wires with several thousand individual conductors are used in the applicator system. Compared to individual thick copper conductors as used in conventional applicators, the skin effect can be reduced or eliminated in the case of high-frequency signals, which leads to a lower power loss in the applicator system according to the improved concept.

The control unit 110 is configured to generate a sequence of electrical pulse signals PS from energy stored in the battery pack 120 for output via the plug connections 130, so that the at least one applicator 200 generates an electromagnetic field when it is connected to the plug connections 130.

With reference to Figure 2, an exemplary sequence of pulse signals PS that may be generated by the control unit 110 is schematically illustrated therein.

For example, each pulse signal PS has a maximum pulse power in the range from 100 kW to 10 MW with a pulse length in the range from 100 µs to 10 µs. The corresponding maximum pulse powers result, for example, from the fact that each pulse signal PS has a maximum current in the range from 100 A to 10 kA. Similarly, the range of the maximum voltage of each pulse signal PS can be in the range from 100 V to 10 kV. For example, the sequence contains 5 to 30 electrical pulse signals PS per second.

For example, each pulse signal PS has a current waveform such that a magnetic field change with a gradient in the order of 1 T/ms results in the applicator 200. This allows the required maximum pulse power to be reliably achieved.

Such an applicator system is suitable and set up for PEMF treatment, for example.

Returning to Figure 1, in some embodiments the applicator system has a passive cooling system. In particular, the applicator system is free of fan cooling and water cooling. Alternatively or additionally, if required, the control unit is cooled by one or more virtually silent fans that generate a noise level of less than 17dB(A). As previously mentioned, the use of high-frequency stranded wire for the coil 210 reduces the power loss compared to conventional coils, so that even at the high maximum power levels, these do not require additional cooling, in particular water cooling. Due to the resulting lower total power in the control unit 110, this can also be designed without a fan or with virtually silent fans. In any case, water cooling can be dispensed with.

The battery pack 120, which supplies the control unit 110 with energy during operation, has a capacity in the range of 200 Wh to 400 Wh, for example. The battery pack 120 is rechargeable, for example, although a corresponding recharging device is not shown for reasons of clarity.

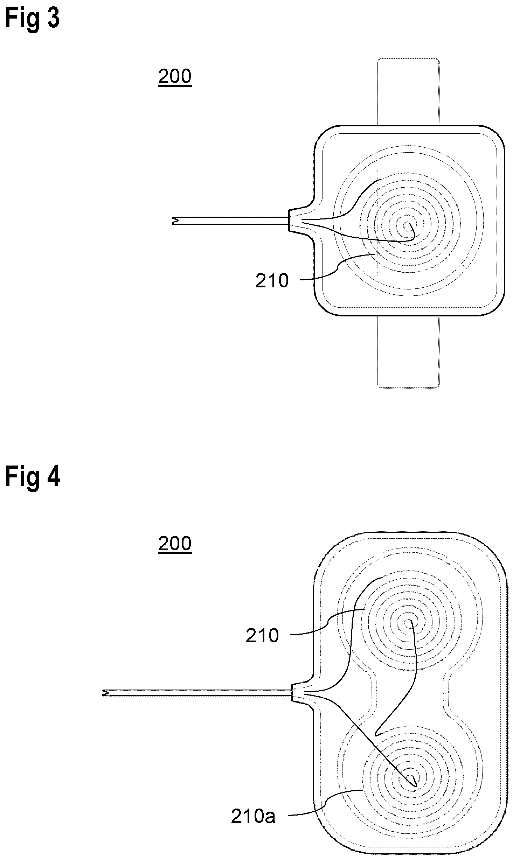

Figures 3 to 6 show various embodiments of applicators for use with the electromagnetic applicator system. Figure 3 shows an example of the systematic representation of an applicator with a single spiral-shaped flat coil in plan view, which is embedded, for example, in a flexible, e.g., rollable, flat body. For example, the flat coil is embedded between two textile surfaces. Figure 3 shows straps with which the applicator 200 can be attached to a part of the body.

It should be noted that the supply line of the applicator 200 is only partially shown. The at least two-pole plug connection is not shown for reasons of clarity.

Figure 4 shows a further embodiment of an applicator which has two flat coils 210, 210a connected in series, similar to the embodiment in Figure 3.

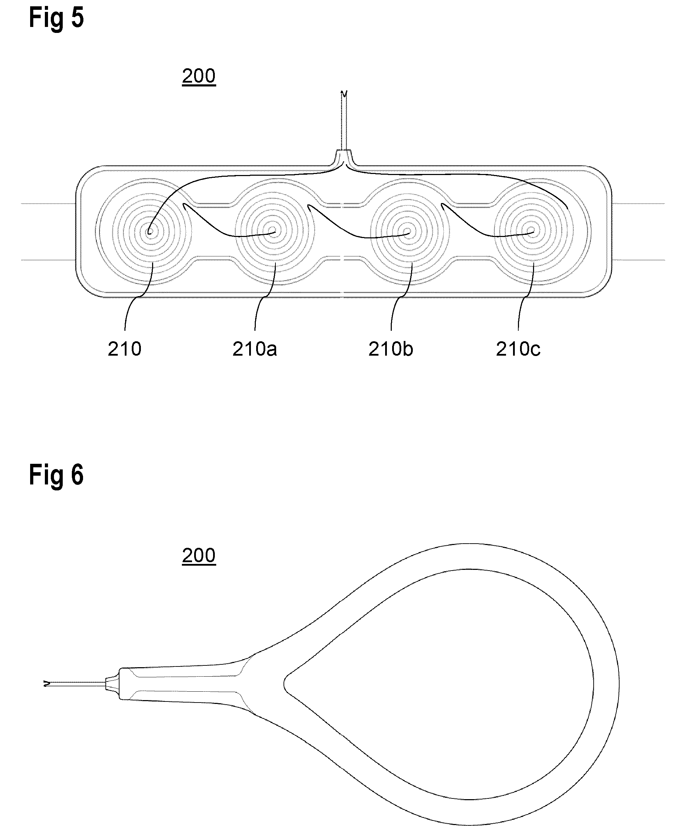

In a further embodiment, Figure 5 shows an applicator 200 with four serially connected flat coils 210, 210a, 210b, 210c. Similar to Figure 3, straps are indicated with which the applicator 200 can be attached to the body.

Figure 6 shows a further embodiment of an applicator which is designed in the form of a sling through which a part of the body, for example an arm or a leg, can be inserted.

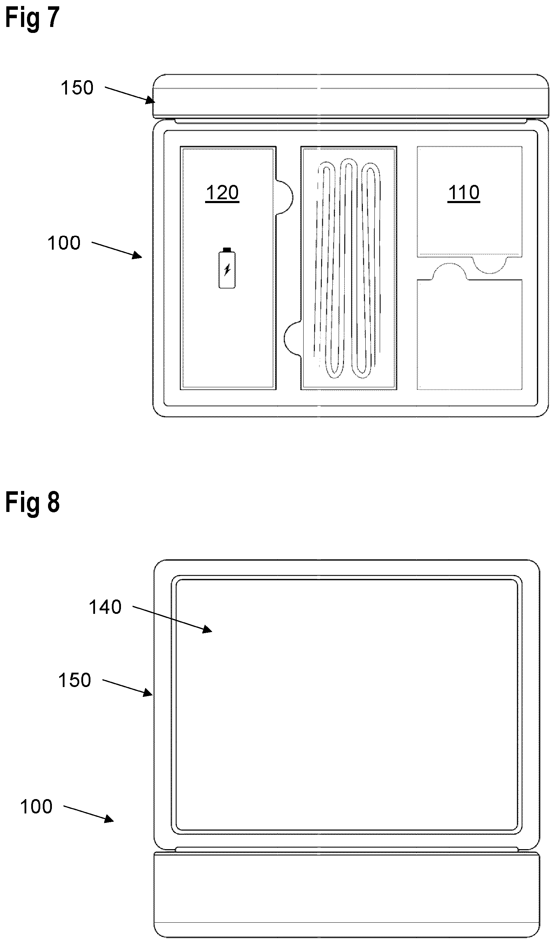

Figures 7 and 8 show illustrations of an embodiment of an electromagnetic applicator system in which the housing 100 is designed as a type of carrying case. Figure 7 shows a top view of the carrying case, in which a lid 150 of the carrying case is open. The control unit 110 and the battery pack 120 can be recognized inside the case, which also has compartments for storing accessories, such as an applicator. For example, the carrying case is about the size of a shoe box.

Figure 8 shows a side view of the case-like housing, in which a control panel 140 is arranged in the lid 150. The control panel is used, for example, to operate the control unit 110 and can, for example, be formed as a touch-sensitive display. This makes it easy for a user to select different programs or directly set parameters of the pulse signals PD or the sequence of pulse signals.

## Claims

1. An electromagnetic applicator system comprising
- a housing (100) which contains a control unit (110), a battery pack (120) for supplying the control unit (110) and plug connections (130) accessible from the outside; and
- at least one applicator (200) which comprises at least one coil (210) which is connectable to the plug connections (130) via a supply line with an at least two-pole plug connection; wherein
- the control unit (110) is configured to generate a sequence of electrical pulse signals (PS) from energy stored in the battery pack (120) for output via the plug connections (130), so that the at least one applicator (200), when connected to the plug connections (130), generates an electromagnetic field; and
- each pulse signal (PS) has a pulse length in the range of 100 µs to 10 ms;
**characterized in that**
- the at least one coil (210) and the supply line are formed with a high-frequency stranded wire; and
- each pulse signal (PS) has a maximum pulse power in the range of 100 kW to 10 MW with the pulse length in the range of 100 µs to 10 ms.

2. The applicator system according to claim 1, wherein each pulse signal (PS) has a maximum current in the range of 100 A to 10 kA and/or a maximum voltage in the range of 100 V to 10 kV.

3. The applicator system according to claim 1 or 2, wherein each pulse signal (PS) has a current waveform such that a magnetic field change with a gradient in the order of magnitude of 1 T/ms results in the applicator (200).

4. The applicator system according to one of claims 1 to 3, wherein the sequence contains 5 to 30 electrical pulse signals (PS) per second.

5. The applicator system according to one of claims 1 to 4, wherein the applicator system is configured for a respective application duration of up to 20 minutes.

6. The applicator system according to one of claims 1 to 5, wherein the applicator system
- comprises passive cooling and is free from fan cooling and water cooling; or
- is free of water cooling and comprises passive cooling and/or fan cooling with one or more fans generating a noise level of less than 17dB(A).

7. The applicator system according to one of claims 1 to 6, wherein the high-frequency stranded wire has several thousand cores.

8. The applicator system according to one of claims 1 to 7, wherein the battery pack (120) has a capacity in the range of 200 Wh to 400 Wh.

9. The applicator system according to one of claims 1 to 8, wherein the housing (100) further comprises a control panel (140) for operating the control unit (110).

10. The applicator system according to claim 9, wherein the housing (100) is formed by a carrying case containing the battery pack (120), the control unit (110) and the control panel (140), and wherein the control panel (140) is arranged in a lid (150) of the carrying case.

## Patentansprüche

1. Elektromagnetisches Applikatorsystem, umfassend
- ein Gehäuse (100), welches ein Steuergerät (110), einen Batteriespeicher (120) zur Versorgung des Steuergeräts (110) und von außen zugängliche Steckanschlüsse (130) enthält; und
- wenigstens einen Applikator (200), der wenigstens eine Spule (210) umfasst, die über eine Zuleitung mit einer wenigstens zweipoligen Steckverbindung an die Steckanschlüsse (130) anschließbar ist; wobei
- das Steuergerät (110) eingerichtet ist, aus im Batteriespeicher (120) gespeicherter Energie eine Folge elektrischer Pulssignale (PS) zur Abgabe über die Steckanschlüsse (130) zu erzeugen, sodass der wenigstens eine Applikator (200), wenn er an die Steckanschlüsse (130) angeschlossen ist, ein elektromagnetisches Feld erzeugt; und
- jedes Pulssignal (PS) eine Pulslänge im Bereich von 100 µs bis 10 ms hat;
**gekennzeichnet dadurch, dass**
- die wenigstens eine Spule (210) und die Zuleitung mittels einer Hochfrequenzlitze gebildet sind; und
- jedes Pulssignal (PS) eine maximale Pulsleistung im Bereich von 100 kW bis 10 MW bei der Pulslänge im Bereich von 100 µs bis 10 ms hat.

2. Applikatorsystem gemäß Anspruch 1, wobei jedes Pulssignal (PS) einen maximalen Strom im Bereich von 100 A bis 10 kA und/oder eine maximale Spannung im Bereich von 100 V bis 10 kV aufweist.

3. Applikatorsystem gemäß Anspruch 1 oder 2, wobei jedes Pulssignal (PS) einen Stromverlauf derart aufweist, dass im Applikator (200) eine Magnetfeldänderung mit einem Gradienten in der Größenordnung von 1 T/ms resultiert.

4. Applikatorsystem gemäß einem der Ansprüche 1 bis 3, wobei die Folge 5 bis 30 elektrische Pulssignale (PS) pro Sekunde enthält.

5. Applikatorsystem gemäß einem der Ansprüche 1 bis 4, wobei das Applikatorsystem für eine jeweilige Applikationsdauer von bis zu 20 Minuten eingerichtet ist.

6. Applikatorsystem gemäß einem der Ansprüche 1 bis 5, wobei das Applikatorsystem
- eine passive Kühlung aufweist und frei ist von einer Lüfterkühlung und einer Wasserkühlung; oder
- frei ist von einer Wasserkühlung und eine passive Kühlung und/oder eine Lüfterkühlung mit einem oder mehreren Lüftern aufweist, die einen Geräuschpegel von weniger als 17dB(A) erzeugen.

7. Applikatorsystem gemäß einem der Ansprüche 1 bis 6, wobei die Hochfrequenzlitze mehrere tausend Adern aufweist.

8. Applikatorsystem gemäß einem der Ansprüche 1 bis 7, wobei der Batteriespeicher (120) eine Kapazität im Bereich von 200 Wh bis 400 Wh aufweist.

9. Applikatorsystem gemäß einem der Ansprüche 1 bis 8, wobei das Gehäuse (100) ferner ein Bedienfeld (140) zur Bedienung des Steuergeräts (110) umfasst.

10. Applikatorsystem gemäß Anspruch 9, wobei das Gehäuse (100) durch einen Koffer gebildet ist, der den Batteriespeicher (120), das Steuergerät (110) und das Bedienfeld (140) enthält, und wobei das Bedienfeld (140) in einem Deckel (150) des Koffers angeordnet ist.

## Revendications

1. Système d'applicateur électromagnétique comprenant
- un boîtier (100) qui contient une unité de commande (110), un bloc-batterie (120) pour alimenter l'unité de commande (110) et des connexions enfichables (130) accessibles de l'extérieur ; et
- au moins un applicateur (200) qui comprend au moins une bobine (210) pouvant être connectée aux connexions enfichables (130) via une ligne d'alimentation avec une connexion enfichable à au moins deux pôles ; dans lequel
- l'unité de commande (110) est configurée pour générer une séquence de signaux électriques impulsionnels (PS) à partir de l'énergie stockée dans le bloc-batterie (120) pour une sortie via les connexions à fiche (130), de sorte que le au moins un applicateur (200), lorsqu'il est connecté aux connexions à fiche (130), génère un champ électromagnétique ; et
- chaque signal d'impulsion (PS) a une durée d'impulsion comprise entre 100 µs et 10 ms ;
**caractérisé en ce que**
- la au moins une bobine (210) et la ligne d'alimentation sont formées avec un fil toronné à haute fréquence ; et
- chaque signal d'impulsion (PS) a une puissance d'impulsion maximale comprise entre 100 kW et 10 MW avec le durée d'impulsion comprise entre 100 µs et 10 ms.

2. Système d'applicateur selon la revendication 1, dans lequel chaque signal d'impulsion (PS) a un courant maximal compris entre 100 A et 10 kA et/ou une tension maximale comprise entre 100 V et 10 kV.

3. Système d'applicateur selon la revendication 1 ou 2, dans lequel chaque signal d'impulsion (PS) a une forme d'onde de courant telle qu'un changement de champ magnétique avec un gradient de l'ordre de grandeur de 1 T/ms se produit dans l'applicateur (200).

4. Système d'applicateur selon l'une des revendications 1 à 3, dans lequel la séquence contient 5 à 30 signaux électriques pulsés (PS) par seconde.

5. Système d'applicateur selon l'une des revendications 1 à 4, dans lequel le système d'applicateur est configuré pour une durée d'applicateur respective allant jusqu'à 20 minutes.

6. Système d'applicateur selon l'une des revendications 1 à 5, dans lequel le système d'applicateur
- comprend un refroidissement passif et est exempt de refroidissement par ventilateur et de refroidissement par eau ; ou
- est exempt de refroidissement par eau et comprend un refroidissement passif et/ou un refroidissement par ventilateur avec un ou plusieurs ventilateurs générant un niveau sonore inférieur à 17 dB(A).

7. Système d'applicateur selon l'une des revendications 1 à 6, dans lequel le fil toronné à haute fréquence comporte plusieurs milliers de conducteurs.

8. Système d'applicateur selon l'une des revendications 1 à 7, dans lequel le bloc-batterie (120) a une capacité comprise entre 200 Wh et 400 Wh.

9. Système d'applicateur selon l'une des revendications 1 à 8, dans lequel le boîtier (100) comprend en outre un panneau de commande (140) pour faire fonctionner l'unité de commande (110).

10. Système d'applicateur selon la revendication 9, dans lequel le boîtier (100) est formé par une mallette de transport contenant le bloc-batterie (120), l'unité de commande (110) et le panneau de commande (140), et dans lequel le panneau de commande (140) est disposé dans un couvercle (150) de la mallette de transport.
